# EUROPEAN PATENT SPECIFICATION

(11) **EP 3 522 778 B1**
(45) Date of publication and mention of the grant of the patent: **30.03.2022**
(21) Application number: 17790692.2
(22) Date of filing: 10.10.2017
(51) Int. Cl.: A61B 5/021

(54) **AN APPARATUS AND METHOD FOR DETERMINING A CALIBRATION PARAMETER FOR A BLOOD PRESSURE MEASUREMENT DEVICE**
VORRICHTUNG UND VERFAHREN ZUR BESTIMMUNG EINES KALIBRIERUNGSPARAMETERS FÜR EINE BLUTDRUCKMESSVORRICHTUNG
APPAREIL ET PROCÉDÉ POUR DÉTERMINER UN PARAMÈTRE D'ÉTALONNAGE POUR UN DISPOSITIF DE MESURE DE PRESSION SANGUINE

(30) Priority: 10.10.2016 EP 16193097
(43) Date of publication of application: 14.08.2019
(73) Proprietor: Koninklijke Philips N.V., 5656 AG Eindhoven (NL)
(72) Inventor: HUIJBREGTS, Laurentia, Johanna, 5656 AE Eindhoven (NL); KAHLERT, Joachim, 5656 AE Eindhoven (NL); SCHMITT, Lars, 5656 AE Eindhoven (NL); HILBIG, Rainer, 5656 AE Eindhoven (NL)
(74) Representative: Philips Intellectual Property & Standards
(86) International application number: PCT/EP2017/075717
(87) International publication number: WO 2018/069261

(56) References cited:
- US-A- 5 755 669
- US-A1- 2002 095 087
- US-A1- 2015 250 394
- US-B1- 9 408 542

## Description

### FIELD OF THE INVENTION

The invention relates to a blood pressure measurement device that is used to obtain measurements of the blood pressure of a subject, and in particular relates to an apparatus and method for determining a calibration parameter for the blood pressure measurement device.

### BACKGROUND TO THE INVENTION

In acute care settings in the hospital, in particular in the intensive care unit (ICU) or operating room (OR), the condition of a patient can change rapidly and immediate action may need to be taken. One vital sign that can provide an indication of the patient's condition is blood pressure (BP). BP is usually measured with an invasive line or with an arm cuff (for example automatically by using the oscillometric technique, which involves analysis of the amplitude of the pressure changes due to the blood pulse, or manually by using the auscultatory technique, which involves the detection of Korotkoff sounds). However, both techniques have significant drawbacks: an invasive line carries a high risk for infection, whereas an arm cuff cannot be used to measure BP continuously, which may mean that there is a delay in identifying that a patient has rapidly deteriorated. Therefore in these scenarios at least, it is desirable to use a blood pressure measurement technique that is non-invasive and continuous (i.e. that allows a blood pressure measurement to be obtained continuously).

Several techniques for obtaining continuous blood pressure measurements in a non-invasive manner exist. For example blood pressure measurements can be obtained from a measurement of pulse wave velocity (PWV), or from features in a photoplethysmography (PPG) signal. In contrast to the invasive line or oscillometric/auscultatory-based methods above, which provide direct measurements of the blood pressure, these blood pressure measurements provide an indirect measurement of blood pressure, and are thus referred to herein as 'surrogate' blood pressure measurements. However, it is necessary for the mathematical relationship that relates blood pressure to a non-invasive continuous surrogate blood pressure measurement to be calibrated regularly in order to obtain accurate measurements of the blood pressure because these surrogate measurements can be influenced by physiological changes other than changes in the blood pressure.

More background information can be found in US 9,408,542 B1, US 2002/0095087 A1, US 2015/0250394 A1, and US 5,755,669 A.

### SUMMARY OF THE INVENTION

To obtain a reliable calibration, measurements of the surrogate and blood pressure at two different blood pressure levels are required (as a minimum). In some cases it can be acceptable to perform the calibration over a significant period of time (e.g. a few hours or days), but in other cases it is desirable to perform the calibration much quicker (e.g. in the case of a blood pressure measurement device used in an ICU or OR). In this if case the measurements need to be made a change in the blood pressure of the subject needs to occur to allow measurements of blood pressure to be made at two or more different levels. US 8,602,997 describes a method for a blood pressure reduction to be automatically induced in the subject to calibrate the considered surrogate parameter, in this case pulse-transit-time. In this method a cuff on the upper arm is inflated to induce changes of blood pressure under the cuff and in the more distal part of the arm (e.g. including the hand and finger).

However, this technique has some disadvantages. For example, the technique can only be used with surrogate blood pressure measurements that measure the blood pressure more distal than the location of the cuff, e.g. the arm/hand/finger when an arm cuff is used, or the leg/foot/toe when a leg cuff is used. Thus, the technique cannot be used to calibrate blood pressure measurements that are obtained using a central site blood pressure measurement technique (e.g. in which blood pressure is determined from the time difference between a pulse in the femoral artery and the carotid artery). A further disadvantage is that as the arm cuff is used to induce blood pressure changes, it cannot at the same time be used to obtain a blood pressure measurement for use in the calibration. Therefore, unless a second cuff is used on the other arm of the subject, the method has to rely on a modelled relationship between the surrogate blood pressure measurement (in this case PAT) and the blood pressure measurement obtained using the cuff.

There is therefore a need for an improved method and apparatus for calibrating measurements of blood pressure obtained by a blood pressure measurement device, and in particular for determining a calibration parameter for the blood pressure measurement device.

According to a first aspect, there is provided an apparatus for determining a calibration parameter for a first blood pressure, BP, measurement device, the apparatus comprising:a control unit that is to be coupled to a first BP measurement device that is for obtaining physiological characteristic measurements of a physiological characteristic of a subject and for determining a blood pressure measurement of the subject from the physiological characteristic measurements, a second BP measurement device that is for obtaining measurements of the blood pressure of the subject and a ventilator that is for providing a controlled flow of gas to the subject, wherein the control unit is configured to:obtain a first physiological characteristic measurement of the subject using the first BP measurement device;control the second BP measurement device to obtain a first BP measurement of the subject;control the ventilator to change one or more properties of the flow of gas to the subject, and thereby change the BP of the subject;analyze a signal from the first BP measurement device to determine when the BP is stable following the change of the one or more properties of the flow of gas;once the BP is determined to be stable following the change of the one or more properties of the flow of gas, obtain a second physiological characteristic measurement of the subject using the first BP measurement device;once the BP is determined to be stable following the change of the one or more properties of the flow of gas, control the second BP measurement device to obtain a second BP measurement of the subject; and determine a calibration parameter for determining BP measurements from physiological characteristic measurements obtained by the first blood pressure measurement device from an analysis of the first physiological characteristic measurement, the second physiological characteristic measurement, the first BP measurement and the second BP measurement.

In some embodiments, the second BP measurement device comprises first and second inflatable cuffs for use on different limbs of the subject, and wherein the control unit is configured to control the second BP measurement device to obtain the first BP measurement of the subject using the first inflatable cuff and to control the second BP measurement device to obtain the second BP measurement of the subject using the second inflatable cuff. This embodiment has an advantage that two cuff-based blood pressure measurements can be taken in a short time interval that are reliable (since the use of cuffs on different limbs means that it is not necessary to allow the blood vessels and blood flow under one of the inflatable cuffs to return to a steady or normal state before taking a BP measurement with the other cuff) and that do not cause significant discomfort for the subject.

In some embodiments, the control unit is further configured to obtain a third BP measurement of the subject from a third physiological characteristic measurement by the first BP measurement device and the determined calibration parameter.

According to a second aspect, there is provided a system comprising the apparatus as described above, the first blood pressure measurement device; the second blood pressure measurement device; and the ventilator.

According to a third aspect, there is provided a computer implemented method of determining a calibration parameter for a first blood pressure measurement device, the method comprising:obtaining a first physiological characteristic measurement of a subject using the first BP measurement device, wherein the first BP measurement device is for obtaining physiological characteristic measurements of a physiological characteristic of the subject and for determining a blood pressure measurement of the subject from the physiological characteristic measurements;controlling a second BP measurement device to obtain a first BP measurement of the subject;controlling a ventilator to change one or more properties of a flow of gas to the subject, and thereby change the BP of the subject;analyzing a signal from the first BP measurement device to determine when the BP is stable following the change of the one or more properties of the flow of gas;once the BP is determined to be stable following the change of the one or more properties of the flow of gas, obtaining a second physiological characteristic measurement of the subject using the first BP measurement device;once the BP is determined to be stable following the change of the one or more properties of the flow of gas, controlling the second BP measurement device to obtain a second BP measurement of the subject; and determining a calibration parameter for determining BP measurements from physiological characteristic measurements obtained by the first blood pressure measurement device from an analysis of the first physiological characteristic measurement, the second physiological characteristic measurement, the first BP measurement and the second BP measurement.

In some embodiments, the steps of obtaining the first physiological characteristic measurement and controlling the second BP measurement device to obtain the first BP measurement are performed at generally the same time. In some embodiments, the steps of obtaining the second physiological characteristic measurement and controlling the second BP measurement device to obtain the second BP measurement are performed at generally the same time. These embodiments provide that there should not be any substantial change in the blood pressure of the subject between the measurements being taken, which enables the measurements to be compared to each other when determining the calibration parameter.

In some embodiments, the second BP measurement device comprises first and second inflatable cuffs for use on different limbs of the subject, and wherein the step of controlling the second BP measurement device to obtain the first BP measurement comprises controlling the second BP measurement device to obtain the first BP measurement of the subject using the first inflatable cuff, and the step of controlling the second BP measurement device to obtain the second BP measurement comprises controlling the second BP measurement device to obtain the second BP measurement of the subject using the second inflatable cuff. This embodiment has an advantage that two cuff-based blood pressure measurements can be taken in a short time interval that are reliable (since the use of cuffs on different limbs means that it is not necessary to allow the blood vessels and blood flow under one of the inflatable cuffs to return to a steady or normal state before taking a BP measurement with the other cuff) and that do not cause significant discomfort for the subject.

In some embodiments, the method further comprises the step of obtaining a third BP measurement of the subject from a third physiological characteristic measurement by the first BP measurement device and the determined calibration parameter.

According to a fourth aspect, there is provided a computer program product comprising a computer readable medium having computer readable code embodied therein, the computer readable code being configured such that, on execution by a suitable computer or processor, the computer or processor is caused to perform any of the methods set out above.

### BRIEF DESCRIPTION OF THE DRAWINGS

Exemplary embodiments of the invention will now be described, by way of example only, with reference to the following drawings, in which:
Fig. 1 is a graph illustrating changes in the heart rate and blood pressure during a Valsalva manoeuvre;
Fig. 2 is a block diagram of a system according to an embodiment that comprises an apparatus according to an embodiment, a first blood pressure measurement device, a second blood pressure measurement device and a ventilator;
Fig. 3 is a graph illustrating an exemplary photoplethysmograph (PPG) signal;
Fig. 4 is an illustration of an exemplary embodiment of an apparatus, first blood pressure measurement device, second blood pressure measurement device and a ventilator in use on a subject; and
Fig. 5 is a flow chart illustrating a method according to an embodiment.

### DETAILED DESCRIPTION OF EMBODIMENTS

As described above, a blood pressure measurement device that uses a 'surrogate' blood pressure measurement technique (i.e. a measurement technique that does not directly measure the blood pressure but infers the blood pressure from measurements of other physiological characteristics) needs to be calibrated using measurements from a blood pressure measurement device that measures blood pressure more directly (for example using the oscillometric technique or using an arterial line). At least two blood pressure measurements/surrogate physiological characteristic measurements are required from each blood pressure measurement device, with those measurements being obtained for different blood pressures, in order to perform the calibration. For this calibration to be completed in a short length of time (e.g. no more than a few minutes, or even within a small number of breathing cycles), it is necessary to induce a change in the blood pressure in the subject so that the measurements at the different blood pressure can be obtained.

US 8,602,997 describes that an inflatable cuff on the upper arm is inflated to modulate the flow resistance in the blood vessels beneath the cuff and thereby to induce changes of blood pressure in the more distal part of the arm (e.g. hands and fingers), but this has disadvantages, as set out above.

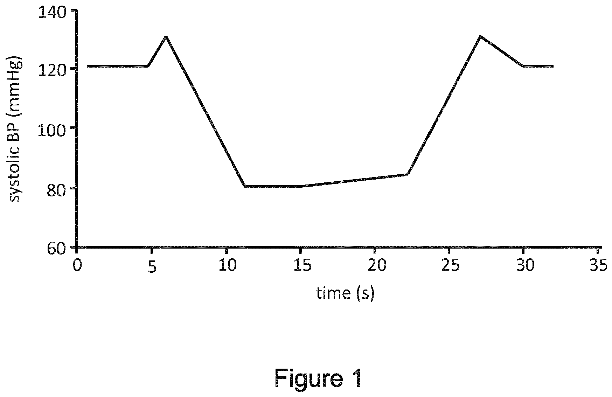

In subjects that are healthy and awake, it is possible to induce blood pressure changes through effort by the subject. For example, the subject could be asked to perform mental or physical exercises, which would induce a change in the blood pressure. Alternatively, the subject could perform a so-called 'Valsalva manoeuvre' in which the air pressure in the lungs is increased by attempting an exhalation while closing the mouth and nose. This manoeuvre tends to temporarily increase the heart rate and decrease the blood pressure in the subject due to cardiopulmonary interactions (i.e. interactions between the lungs/ventilation and heart/circulation). Fig. 1 shows how the systolic blood pressure changes during a Valsalva manoeuvre. In particular, it can be seen that shortly after the start of the Valsalva manoeuvre (which is at t=0) the blood pressure increases slightly but then falls significantly (e.g. by around 50 mmHg) over a period of around 5 seconds. The blood pressure then plateaus from around t=11 to around t=22 where it starts to increase and return to the pre-Valsalva manoeuvre level. On a physiological level, the strong positive pressure in the thorax during the Valsalva manoeuvre impedes the venous return flow and lowers the atrial refilling which consequently causes a reduced stroke volume. The reduced stroke volume is directly correlated to a drop in the systemic blood pressure.

As another alternative, the subject could perform a sigh. Sighing (inhaling a larger-than-normal volume of air) has several beneficial effects. It opens up the alveoli, releases surfactants which form a layer in the alveoli important for their elasticity, and the respiration-induced stretching of the lungs activates mechanoreceptors, which leads to vessel dilatation and thereby to lowering of the blood pressure. Another possibility is the so-called Mueller Manoeuvre in which the subject attempts to inhale against a closed airway.

These voluntary breathing manoeuvres, and even involuntary breathing manoeuvres, such as yawning, coughing and sighing, change the transmural pressure on the blood vessels in the chest and change the venous return flow. These pressure changes are sensed by mechanical receptors in the central veins, the right atrium and the pulmonary vessels. The receptors are baroreceptors and stretch receptors which invoke a neural response. The neural response causes a dynamic blood pressure change.

Although these breathing manoeuvres can be used to induce a change in the blood pressure, since patients in the ICU or OR are often unconscious, they are not able to perform these manoeuvres. Even for subjects that are conscious, they may not perform the breathing manoeuvre correctly (or be able to perform them correctly).

In view of these difficulties, embodiments of the invention provide that a ventilator is controlled to change a flow of gas provided to the subject and thereby induce/invoke a change in the blood pressure. In some embodiments, the ventilator can be controlled to change the pressure and/or flow rate of the gas provided to the subject. In some embodiments, this change in the pressure and/or flow rate can be used to mimic a Valsalva manoeuvre in the subject. In some embodiments, the ventilator can also or alternatively be controlled to change the composition of the gas provided to the subject, since it is known that the blood pressure of a subject can be affected by the amount of oxygen in the inhaled air (which is known from, for example, "Changes in 24 h ambulatory blood pressure and effects of angiotensin II receptor blockade during acute and prolonged high-altitude exposure: a randomized clinical trial" by Parati et al. in European Heart Journal (2014) 35, 3113-3121). Physiological characteristic measurements can be taken by the blood pressure measurement device that uses a surrogate measurement technique and blood pressure measurements can be taken by the blood pressure measurement device that measures the blood pressure more directly before and after this induced change, and these measurements are used to calibrate the relationship between the surrogate blood pressure measurements and blood pressure, and thus enable accurate blood pressure measurements to be obtained from subsequent measurements of the surrogate physiological characteristic. The use of the ventilator thus allows a blood pressure change to be induced in a subject that is unconscious, without interfering with a clinical workflow (for example in an ICU or OR scenario). Of course, it will be appreciated that the ventilator can also be used to induce a blood pressure change in a subject that is conscious.

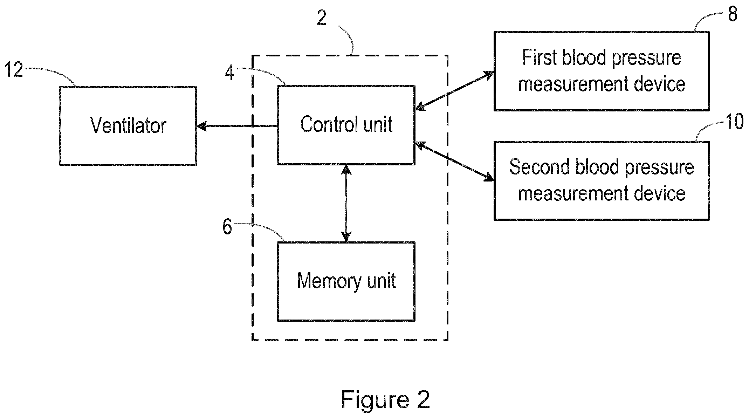

An embodiment of an apparatus 2 for determining a calibration parameter for a first blood pressure measurement device is shown in Fig. 2. The apparatus 2 comprises a control unit 4 and a memory unit 6. The apparatus 2 is shown as part of a system in which the apparatus 2 is coupled (e.g. connected) to a first blood pressure measurement device 8 and a second blood pressure measurement device 10. The first blood pressure measurement device 8 can measure one or more physiological characteristics of the subject that can be processed (using one or more calibration parameters) to determine the blood pressure of the subject, and the physiological characteristic measurements can be provided to the apparatus 2, and specifically to the control unit 4. It will be understood that the first blood pressure measurement device 8 does not obtain a direct measurement of blood pressure, but instead the first blood pressure measurement device 8 measures a physiological characteristic that is a surrogate for blood pressure, in the sense that blood pressure can be derived from the surrogate physiological characteristic. After calibration, the first blood pressure measurement device 8 may process a physiological characteristic measurement to determine a blood pressure measurement, or the first blood pressure measurement device 8 may provide a subsequent physiological characteristic measurement to the control unit 4, and the control unit 4 can determine the blood pressure measurement from the physiological characteristic measurement and the calibration parameter. The second blood pressure measurement device 10 measures the blood pressure of the subject, and the measurements of blood pressure can be provided to the control unit 4. The control unit 4 can control the taking or triggering of physiological characteristic measurements by the first blood pressure measurement device 8 and blood pressure measurements by the second blood pressure measurement device 10.

The apparatus 2 can be coupled to the first blood pressure measurement device 8 and the second blood pressure measurement device 10 using any suitable means or type of connection, for example using wires, or using a wireless connection (e.g. a short or long range communication protocol such as Bluetooth, Wi-Fi, 3G or 4G cellular communications, etc.).

As described in more detail below, the apparatus 2 uses measurements of blood pressure by the second blood pressure measurement device 10 to determine one or more calibration parameters that relate the measurements of the physiological characteristic by the first blood pressure measurement device 8 to blood pressure.

The first blood pressure measurement device 8 and second blood pressure measurement device 10 are different types of devices that measure the blood pressure using different techniques.

The first blood pressure measurement device 8 is a type of measurement device that requires regular calibration (e.g. of the order of a few minutes, every hour, or when a physiological characteristic changes significantly (e.g. by more than a threshold amount)) in order to provide accurate, or sufficiently accurate, measurements of blood pressure from measurements of a (non-blood pressure) physiological characteristic. A mathematical function is used to relate the surrogate physiological characteristic measurement to blood pressure, and this function includes one or more factors and/or constants. The values of these factors and/or constants are determined in the calibration procedure. These factors and/or constants are referred to herein as "calibration parameters". Thus the calibration of the first blood pressure measurement device 8 is provided by determining (values for) one or more calibration parameters, that are used as part of the mathematical function to convert the physiological characteristic measurement obtained by the device 8 to the subject's blood pressure. After calibration, the first blood pressure measurement device 8 is preferably able to provide a continuous or near continuous (e.g. every second, every minute, or every heart beat) measurement of blood pressure, and also preferably obtains the measurement non-invasively.

For example, the surrogate physiological characteristic measurement used to determine blood pressure can be a pulse arrival time (PAT) measurement, which makes use of the time difference between the R-peak in an electrocardiogram (ECG) signal and the time of arrival of a pulse in a finger, which can be measured using a photoplethysmography (PPG) sensor or an accelerometer.

As another example, the first blood pressure measurement device 8 can use pulse wave velocity (PWV) as the surrogate physiological characteristic measurement that is used to determine blood pressure. PWV can be measured as the time difference between the arrival of a pulse at two different locations in the body, e.g. in a femoral artery and in the carotid artery, which is known as the Pulse Transit Time (PTT). The PWV can be measured using a sensor at each location, and the sensor can be, for example, a photoplethysmography (PPG) sensor or an accelerometer.

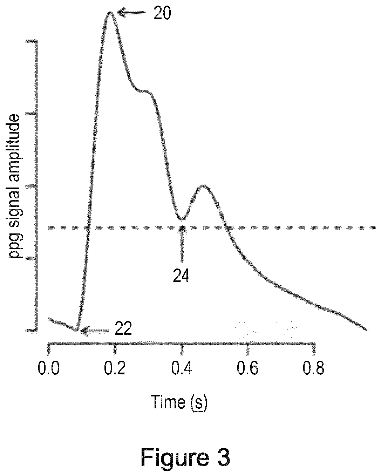

Alternatively the physiological characteristic can comprise various signal characteristics that can be determined from a PPG waveform (signal) and blood pressure can be obtained from a combination of features in the PPG signal. An exemplary PPG signal is shown in Fig. 3, and the features that can be used to determine blood pressure include any one or more of the amplitude of the highest peak 20 in the PPG signal, the DC value of the PPG signal, the slope or gradient of a line connecting the foot 22 of the pulse with the peak 20 of the pulse, or the location (in time) of the dicrotic notch 24.

Alternatively, the physiological characteristic can comprise features of a pressure signal, palpated at the periphery (e.g. a finger), could be used to determine the blood pressure. For example, the air pressure in a finger cuff or air bladder, which is pressurized at a peripheral arterial location, can be measured and features extracted.

The above types of blood pressure measurement devices and their construction and method of operation will be well known to those skilled in the art, and thus detailed explanations are not provided in this document.

In contrast to the first blood pressure measurement device 8, the second blood pressure measurement device 10 is a type of measurement device that does not require regular calibration using measurements from another blood pressure measurement device in order to provide accurate, or sufficiently accurate, measurements of blood pressure. The second blood pressure measurement device 10 is typically a device that can be used to obtain intermittent measurements of blood pressure, although one that does so with relatively high accuracy. The second blood pressure measurement device 10 preferably uses a so-called 'direct' measurement to determine the blood pressure, although the second blood pressure measurement device 10 is preferably not a device that obtains the measurement invasively. For example, the second blood pressure measurement device 10 can comprise an inflatable cuff and a sound sensor that is used to detect Korotkoff sounds as the cuff is inflated and/or deflated (this type of device uses the auscultatory technique).

Alternatively, the second blood pressure measurement device 10 can use the oscillometric technique in which the device 10 comprises an inflatable cuff and a pressure sensor and the pressure in the inflatable cuff is measured and the oscillations in the pressure analyzed to determine the blood pressure.

As another alternative, the second blood pressure measurement device 10 can be an invasive device that is implanted in the body, for example in an artery, and that directly measures the blood pressure. It will be appreciated that although these types of devices can provide continuous or semi-continuous measurements of blood pressure, they may be subject to battery constraints, and thus the implanted device may be used in a power saving mode to maximize the battery life and thus may only be used to obtain the blood pressure measurements required for calibrating the first blood pressure measurement device 8.

In some embodiments, the second blood pressure measurement device 10 can be a type of device that is self-calibrating (i.e. a device that does require calibrating to obtain accurate measurements, but does not require the use of another blood pressure measurement device to do so). Examples include volume-clamp based blood pressure measurement devices, which use a cuff on a finger or other peripheral part of the body of the subject and a PPG sensor. It will be appreciated that although these types of devices can provide continuous or semi-continuous measurements of blood pressure, they may cause discomfort to the subject by the continuous pressure on the finger, and thus the volume-clamp device may be used in a 'comfort' mode to minimize the patient discomfort and thus may only be used to obtain the blood pressure measurements required for calibrating the first blood pressure measurement device 8, while the cuff may be deflated the rest of the time.

In other embodiments, the second blood pressure measurement device 10 can be based on applanation tonometry.

These types of blood pressure measurement devices and their construction and method of operation will be well known to those skilled in the art, and thus detailed explanations are not provided in this document.

As described in more detail below, the second blood pressure measurement device 10 is used to obtain two measurements of blood pressure in order to determine a calibration parameter for the first blood pressure measurement device 8. These measurements may be obtained in a relatively short space of time (e.g. a few seconds or up to a minute or two apart). Where the second blood pressure measurement device 10 uses a cuff to obtain the blood pressure measurements, it can be difficult to obtain two reliable blood pressure measurements in this period of time as it can take several minutes for the blood flow to return to a normal state after a cuff is deflated. Thus, in some embodiments, to avoid this problem, the second blood pressure measurement device 10 can comprise means to enable measurements to be obtained from two different sites on the subject's body (e.g. from both arms). Thus, in some embodiments the second blood pressure measurement device 10 comprises two inflatable cuffs and respective sensors (e.g. sound sensors in the case of an auscultatory measurement and pressure sensors in the case of an oscillometric measurement) that are used on different limbs (e.g. different arms), with one cuff being used to obtain one of the blood pressure measurements and the other cuff being used to obtain the other blood pressure measurement. It will be appreciated that in embodiments in which the second blood pressure measurement device 10 comprises means to enable measurements to be obtained from two different sites on the subject's body, the second blood pressure measurement device 10 may comprise two separate blood pressure measurement devices. This has several advantages in that the calibration process is less uncomfortable for the subject (since using the same cuff twice in a short space of time can be painful), the calibration process is more reliable since there is no influence on the second measurement from the first measurement, and the measurements can be performed quicker, since it is not necessary to complete the deflation of one cuff before starting the inflation of the other.

The system can also comprise a ventilator 12 that is used to provide a controlled flow of gas (e.g. air) to a subject, and in particular to the airway of the subject. The apparatus 2 (and in particular the control unit 4) can be coupled to the ventilator 12. The coupling between the apparatus 2 and the ventilator 12 can be made using any suitable means or type of connection. The ventilator 12 can comprise a patient interface device, such as a face mask, nasal mask, mouthpiece or tracheal tube, that is used to provide or convey a controlled flow of gas using a pump, a compressor or a valve to the subject.

The ventilator 12 can be configured to selectively provide a flow of gas at one or more flow rates, at one or more pressures and/or at one or more compositions (e.g. with different levels of certain gases, such as oxygen, etc.), under control of the control unit 4. Thus, the control unit 4 can output a control signal to the ventilator 12 to set, change or adjust one or more properties of the gas flow provided by the ventilator 12, such as the flow rate, pressure and/or composition of the gas flow. As noted above, this change in the flow rate, pressure and/or composition of the gas flow is used to induce a change in the blood pressure of the subject, and for example in a subject that is sedated or sleeping, although it will be appreciated that the techniques can also be used on a subject that is awake.

In some embodiments, the ventilator 12 is a device that is used to assist the subject's breathing, for example by providing air flow to and/or away from the subject to stimulate or supplement the subject's own breathing motions (inhalations and/or exhalations). In other embodiments, the ventilator 12 is a device that is used to provide air flow at a pressure that is higher than ambient pressure, for example a device that is used to provide a continuous positive airway pressure (CPAP) or a bi-level positive airway pressure (BPAP). As is known, CPAP and BPAP are well-known therapies for subjects with sleep apnea, where the positive pressure (or pressures in the case of BPAP) helps keep the subject's airways open while they sleep.

In some embodiments, the ventilator 12 can be configured to induce a sigh (i.e. to cause the subject to inhale a larger-than-normal volume of air) at regular intervals or at a specified time. This is often used by ventilators 12 that are used for ventilating a subject for several hours or more. Due to the interactions between the pulmonary and circulatory system, these sighs will also lead to a change in the blood pressure (especially in the pulmonary blood pressure, but also to a lesser extent in the systemic blood pressure). The blood pressure change during sighing will be stronger than during tidal breathing in rest or in sleep. Those skilled in the art will be aware that various conventional ventilators include a 'sigh' setting or function.

In some embodiments the ventilator 12 may be configured to induce the subject to perform a Valsalva manoeuvre, for example by closing a valve during an exhalation by the subject (i.e. temporarily stopping the flow of gas). In other embodiments the ventilator 12 may be configured to induce the subject to perform a Mueller manoeuvre by closing a valve during inspiration (i.e. temporarily stopping the flow of gas). For example in sleeping and spontaneously breathing subjects, the inspiratory airflow in a CPAP ventilator 12 can be closed by a valve. Due to the breathing effort in the sleeping subject, a negative pressure is induced. The negative pressure pulse creates a pulsed change of the transmural pressure. This negative pressure pulse causes two effects; first it invokes the neural reflex and secondly it increases the venous return flow. Physiologically, both contribute to a change of the blood pressure. This can be considered to be a Mueller manoeuvre in tidal breathing.

In embodiments where the ventilator 12 is controlled to change the composition of the gas provided to the subject, the ventilator 12 can be controlled to increase the oxygen content (i.e. increase the percentage of oxygen in the air), which will induce a decrease in blood pressure of the subject. Alternatively the ventilator 12 can be controlled to decrease the oxygen content (i.e. decrease the percentage of oxygen), which will induce an increase in the blood pressure of the subject. Those skilled in the art will appreciate that the quantity of other components of the gas (e.g. air) can be increased or decreased in order to change the blood pressure of the subject.

The control unit 4 controls the operation of the apparatus 2, for example controlling the triggering of the collection of physiological characteristic measurements by the first blood pressure measurement device 8 and the blood pressure measurements by the second blood pressure measurement device 10, as well as controlling the operation or settings of the ventilator 12. The control unit 4 can also control other functions and operations of the apparatus 2. The control unit 4 can be implemented in numerous ways, with software and/or hardware, to perform the various functions required. The control unit 4 may comprise one or more microprocessors that may be programmed using software to perform the required functions. The control unit 4 may be implemented as a combination of dedicated hardware to perform some functions and a processor (e.g., one or more programmed microprocessors and associated circuitry) to perform other functions. Examples of processing components that may be employed in various embodiments of the present disclosure include, but are not limited to, conventional microprocessors, application specific integrated circuits (ASICs), and field-programmable gate arrays (FPGAs).

In various implementations, the control unit 4 may be associated with one or more storage media, shown as memory unit 6 in Fig. 2. The memory unit 6 can be part of the control unit 4, or it can be a separate component in the apparatus 2 that is connected to the control unit 4. The memory unit 6 can comprise any suitable or desired type of volatile or non-volatile computer memory such as RAM, PROM, EPROM, and EEPROM. The memory unit 6 can be used for storing computer program code that can be executed by the control unit 4 to perform the method described herein. The memory unit 6 can also be used to store signals or measurements from the first blood pressure measurement device 8 and the second blood pressure measurement device 10, and/or information relating to the calibration of the first blood pressure measurement device 8.

In some embodiments, the first blood pressure measurement device 8 and the second blood pressure measurement device 10 are separate from the apparatus 2 (i.e. they are separate devices). As noted above, the apparatus 2 can be coupled to the first blood pressure measurement device 8 and the second blood pressure measurement device 10 wirelessly or using wires. In these embodiments the apparatus 2 can be implemented by a personal electronic device such as a smartphone, tablet computer, laptop computer or desktop computer, or electronic device present in a clinical environment, such as a bedside monitor, patient monitoring system, sensor array, etc. In some embodiments, the apparatus 2 can be implemented in a computer or server that is remote from the first blood pressure measurement device 8 and the second blood pressure measurement device 10 (e.g. the apparatus 2 could be located in the cloud, i.e. accessible via the Internet).

In other embodiments, the first blood pressure measurement device 8 is part of (i.e. integral with) the apparatus 2, and the apparatus 2 can be coupled (e.g. using wires or wirelessly) to the second blood pressure measurement device 10.

It will be appreciated that Fig. 2 only shows the components required to illustrate various embodiments of the apparatus 2, and in a practical implementation the apparatus 2 will comprise additional components to those shown. For example, the apparatus 2 may comprise a battery or other power supply for powering the apparatus 2, a communication module for enabling the blood pressure measurements or determined calibration parameters for the blood pressure measurements by the first blood pressure measurement device 8 to be communicated to another device, e.g. a remote computer (e.g. that stores health parameter measurement records for the subject) and/or one or more user interface components that allow the subject or another user to interact and control the apparatus 2. As an example, the one or more user interface components could comprise a switch, a button or other control means for activating and deactivating the apparatus 2 and/or calibration parameter determination process. The user interface components can also or alternatively comprise a display or other visual indicator for providing information to the subject and/or other user about the operation of the apparatus 2, including displaying information on a determined blood pressure.

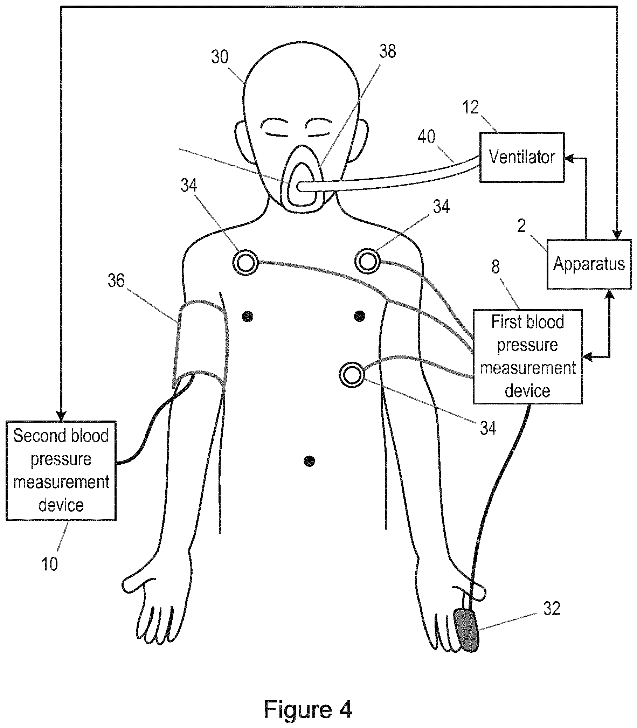

Fig. 4 is an illustration of an exemplary embodiment of an apparatus 2, a first blood pressure measurement device 8, a second blood pressure measurement device 10 and a ventilator 12 in use on a subject 30. In this exemplary embodiment, the first blood pressure measurement device 8 measures pulse arrival time (PAT) as the surrogate physiological characteristic measurement and thus comprises a PPG sensor 32 that obtains a PPG signal from a finger of the subject 30 and a plurality of electrodes 34 that are positioned on the chest or thorax of the subject 30 to obtain an ECG signal from the subject 30. The PAT is determined from the timing of a pulse in the PPG signal and the corresponding R-peak in the ECG signal. The blood pressure of the subject can be determined from the PAT and the calibration parameter(s).

In this example the second blood pressure measurement device 10 uses the auscultatory method to determine blood pressure, and thus comprises an inflatable cuff 36 and a microphone or other sound sensor (not shown) that measures the sounds generated by the pulsatile blood flow through the arteries in the arm, and in particular Korotkoff sounds. The blood pressure measurements by the second blood pressure measurement device 10 obtained using the auscultatory method are used to calibrate (or rather determine a calibration parameter for) the measurements of blood pressure obtained from the PAT measurements by the first blood pressure measurement device 8. As a result, the measurements by the second blood pressure measurement device 10 are referred to herein as 'calibration measurements'.

The ventilator 12 comprises a patient interface device 38 in the form of a face mask that is placed over the mouth and nose of the subject 30, and that is connected to the ventilator 12 via tube or hose 40. The ventilator 12 is used to induce a blood pressure change in the subject.

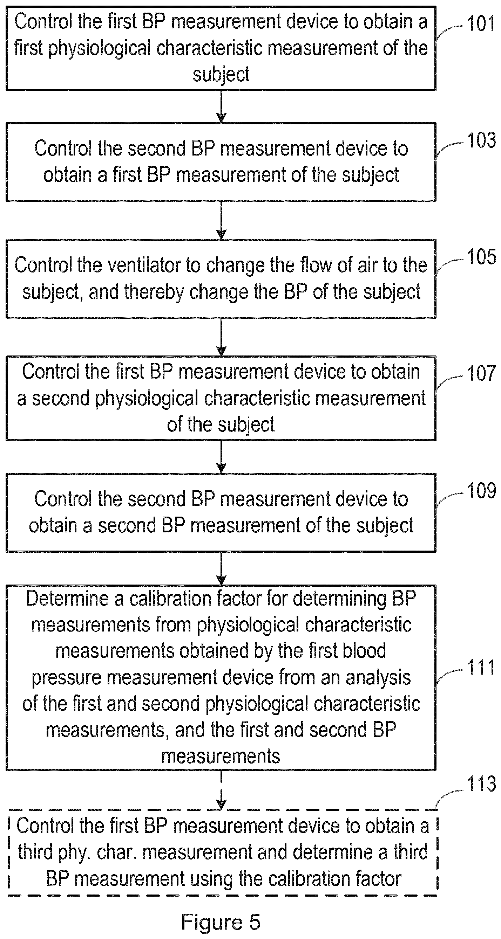

The flow chart in Fig. 5 illustrates a method of determining a calibration parameter for the first blood pressure measurement device 8 in accordance with an embodiment. This method can be implemented or performed by the control unit 4. Since the first blood pressure measurement device 8 is to be used to continuously or semi-continuously to measure a physiological characteristic in the subject 30 and requires calibration before sufficiently accurate blood pressure measurements can be obtained from those physiological characteristic measurements, the method of Fig. 5 can be performed when the first blood pressure measurement device 8 is first activated. The method of Fig. 5 can also be performed periodically in order to recalibrate the first blood pressure measurement device 8 (e.g. every few minutes or every hour, etc.) or as required (e.g. when the physiological characteristic being measured by the first blood pressure measurement device 8 has changed by more than a threshold amount).

In step 101, a measurement of the surrogate physiological characteristic of the subject is obtained using the first blood pressure measurement device 8. This step may comprise the control unit 4 outputting a suitable control or trigger signal to the first blood pressure measurement device 8 to cause the first blood pressure measurement device 8 to perform a physiological characteristic measurement, or, since the first blood pressure measurement device 8 may be continuously measuring the physiological characteristic, this step may comprise the control unit 4 receiving or obtaining the most recent physiological characteristic measurement from the first blood pressure measurement device 8. This physiological characteristic measurement is referred to as the 'first' physiological characteristic measurement.

In step 103, the second blood pressure measurement device 10 is controlled by the control unit 4 to obtain a blood pressure measurement of the subject 30 (for example by the control unit 4 outputting a suitable control or trigger signal to the second blood pressure measurement device 10). This blood pressure measurement is referred to as the 'first' blood pressure measurement or a 'first calibration measurement'. Steps 101 and 103 can be performed at the same time, or performed (in either order) sufficiently close in time that no change in the blood pressure of the subject 30 will have occurred between the taking of the first physiological characteristic measurement and the first blood pressure measurement. For example the first physiological characteristic measurement and the first blood pressure measurement can be obtained during the same breathing cycle (i.e. within the same breath). Alternatively, for subjects with a stable blood pressure (i.e. where the blood pressure is relatively constant over time), the time between the first physiological characteristic measurement and the first blood pressure measurement can be a few seconds, or even a few minutes. It will be appreciated that where the first blood pressure measurement device 8 measures the physiological characteristic at a more distal site than a cuff-based second blood pressure measurement device 10, the first blood pressure measurement device 8 should not obtain the first physiological characteristic measurement while the arm cuff is being inflated or deflated. In that case, the first physiological characteristic measurement could be obtained before the second blood pressure measurement device 10 starts to take the first blood pressure measurement, or at some time after the deflation of the cuff has finished and the blood flow has had a chance to return to a stable level.

Next, in step 105, the control unit 4 controls the ventilator 12 to change a property of the flow of gas to the subject 30, and thereby induce a change in the blood pressure of the subject 30. The property that is changed can be the gas flow rate, the pressure of the gas flow, and/or the composition of the gas (e.g. the oxygen content). It will be appreciated that it does not matter whether the blood pressure change is an increase or decrease. In particular embodiments the control unit 4 can control the ventilator 12 to change the air flow rate (i.e. the velocity of the air flow) and/or the pressure of the air flow. The change in flow rate can be an increase or decrease. For example the flow rate can be increased of the order of three to four times compared to normal ventilator operation. Where step 105 comprises changing the pressure of the gas flow, the pressure change can be of the order of 5-10 millibar (500-1000 Pascal). The change in the air flow rate and/or pressure can be used to cause the subject to perform (i.e. mimic) a Valsalva manoeuvre. In an alternative embodiment, for example where the ventilator 12 is a CPAP ventilator, step 105 can comprise changing the amount of positive pressure (e.g. of the order of 5-10 millibar). In an alternative embodiment, the control unit 4 can control the ventilator 12 to induce a sigh in the subject 30 (i.e. the ventilator 12 provides a larger-than-normal volume of air in a particular inhalation for the subject 30). When the ventilator 12 is used to induce a sigh in the subject, step 105 can comprise increasing the flow rate to three or four times higher than that used for normal tidal breathing. It will be appreciated that the conventional sigh function in the ventilator 12 can be used in this step.

In some embodiments, where the subject does not otherwise require support from the ventilator 12, the ventilator 12 can be deactivated until step 105 is to be performed, in which case step 105 comprises activating the ventilator 12 to provide a gas flow at a particular pressure, flow rate and/or composition to induce a change in the BP of the subject. It will be appreciated that in this embodiment, step 105 results in the flow of gas being changed from no gas flow to gas with a non-zero flow rate and pressure.

After controlling the ventilator 12 to induce a change in the blood pressure of the subject 30, the control unit 4 obtains another measurement of the physiological characteristic using the first blood pressure measurement device 8 (step 107). This physiological characteristic measurement is referred to as the 'second' physiological characteristic measurement. As with step 101, since the first physiological characteristic measurement device 8 is monitoring the physiological characteristic continuously or semi-continuously, step 107 may comprise the control unit 4 obtaining a current physiological characteristic measurement from the first blood pressure measurement device 8, rather than the control unit 4 explicitly requesting a physiological characteristic measurement at that time. Alternatively however, this step can comprise the control unit 4 controlling the first blood pressure measurement device 8 to make a measurement of the physiological characteristic.

The control unit 4 also controls the second blood pressure measurement device 10 to obtain another blood pressure measurement of the subject 30 (step 109). This blood pressure measurement is referred to the 'second' blood pressure measurement or a 'second' calibration measurement'. As noted above, in some embodiments the second blood pressure measurement device 10 can include means to enable the second blood pressure measurement device 10 to obtain blood pressure measurements from two different sites on the subject's body. For example the second blood pressure measurement device 10 can comprise two inflatable cuffs, in which case step 109 can comprise obtaining the second blood pressure measurement using a different cuff to that used in step 103 to obtain the second blood pressure measurement.

As with the first physiological characteristic measurement and the first blood pressure measurement, steps 107 and 109 can be performed at the same time, or they can be performed sufficiently close in time (in either order) that no change in the blood pressure of the subject 30 will have occurred between the taking of the second physiological characteristic measurement and the second blood pressure measurement. The other requirements described above for the relative timing of the first physiological characteristic measurement and the first blood pressure measurement can apply also to the relative timing of the second physiological characteristic measurement and the second blood pressure measurement.

As the blood pressure change induced by the ventilator 12 only occurs for a relatively short time (e.g. a few seconds) steps 107 and 109 are performed shortly after step 105 is performed, although it will be appreciated that the change in the blood pressure does not occur instantly when the ventilator 12 changes the flow of air, and thus the control unit 4 allows a sufficient amount of time to elapse from the change in the air flow before triggering the first blood pressure measurement device 8 and the second blood pressure measurement device 10 to obtain the second physiological characteristic measurement and the second blood pressure measurements respectively. The timing of the second physiological characteristic measurement and the second blood pressure measurement is described in more detail below.

After step 109, the control unit 4 determines one or more calibration parameters for the first blood pressure measurement device 8 from an analysis of the first physiological characteristic measurement, the second physiological characteristic measurement, the first blood pressure measurement and the second blood pressure measurement (step 111). That is, the control unit 4 determines one or more calibration parameters that are to be applied to subsequent physiological characteristic measurements by the first blood pressure measurement device 8 in order to provide accurate (or sufficiently accurate) measurements of the blood pressure of the subject 30. Typically that is done by means of regression, i.e. a modelled functional relation or other mathematical relationship that maps a surrogate physiological characteristic measurement to blood pressure is fitted to the measured surrogate measurements (i.e. the measurements of the physiological characteristics obtained in steps 101 and 107) and blood pressure measurements from the second blood pressure measurement device 10. As an outcome of the fitting (mapping), the values for the one or more calibration parameters are obtained.

Once the one or more calibration parameters have been determined in step 111, the control unit 4 or the first blood pressure measurement device 8 (as appropriate) can use the one or more calibration parameters to determine blood pressure measurements of the subject 30. This is shown as optional step 113 in Fig. 5. In particular, in step 113, the control unit 4 can control or trigger the first blood pressure measurement device 8 to obtain at least one further physiological characteristic measurement (which is referred to as the 'third' physiological characteristic measurement. The one or more calibration parameters determined in step 111 are used in the mathematical function that relates the third physiological characteristic measurement (e.g. PAT) to blood pressure. This blood pressure measurement is referred to as a 'calibrated measurement' of blood pressure. Step 113 can be repeated to provide a continuous or semi-continuous measurement of the blood pressure of the subject 30.

After the one or more calibration parameters have been determined in step 111 (or perhaps once the second physiological characteristic measurement and second blood pressure measurement have been obtained in steps 107 and 109), the second blood pressure measurement device 10 can be deactivated (i.e. the cuff deflated, if the second blood pressure measurement device 10 comprises a cuff), since it is only required for determining the one or more calibration parameters. The second blood pressure measurement device 10 may subsequently be reactivated or used when a calibration parameter is to be updated.

Likewise, after step 111 or perhaps after steps 107/109, the ventilator 12 can be controlled to change the air flow to the subject 30 so that the flow of air does not induce, or no longer induces, the change in blood pressure. Thus, for example, the ventilator 12 can be controlled so that the air flow rate and/or pressure are returned to the air flow rate and/or pressure that were used prior to step 105. Where the subject 30 does not otherwise require the use of a ventilator 12, the control unit 4 may deactivate the ventilator 12 (i.e. so the air flow rate and pressure of the air flow are zero). In this case a patient interface device 38 that is used to provide the flow of air to the subject 30 can be removed from the face of the subject 30. When a calibration parameter is to be updated, the patient interface device 38 can be reapplied to the subject 30 and the ventilator 12 reactivated.

In embodiments where the ventilator 12 is used to regularly induce a sigh in the subject 30, the method of Fig. 5 can be performed to make use of these regular sighs to perform the calibration. Thus, when a sigh is to be performed as part of the conventional sighing cycle, steps 101 and 103 can be timed to be performed prior to a sigh, and steps 107 and 109 can be timed to be performed after the sigh. In this case, step 105 comprises the ventilator 12 being controlled to cause a sigh in accordance with the ongoing sighing cycle. In this embodiment, the calibration procedure of Fig. 5 can be performed each time a sigh will occur, or only for certain sighs (e.g. every other sigh, every fifth sigh, etc.).

In some embodiments, to make sure that the first blood pressure measurement and the second blood pressure measurement are reliable, the control unit 4 can analyze the physiological characteristic measurement made by the first blood pressure measurement device 8 to determine if the blood pressure is stable (i.e. constant) at the time of the blood pressure measurement by the second blood pressure measurement device 10. In particular, the control unit 4 can analyze the measurement of the physiological characteristic, for example the PWV, the PAT, the PTT, or analyze relevant characteristics of the measurement signal itself (i.e. the characteristics relevant to determining the blood pressure), e.g. the PPG signal, the accelerometer signal or the ECG signal, to determine if the relevant characteristics are constant or substantially constant (i.e. they do not vary by more than a threshold amount) during the period in which the second blood pressure measurement device 10 is measuring the blood pressure. If the physiological characteristics are not constant (or not substantially constant), the control unit 4 can discard that blood pressure measurement by the second blood pressure measurement device 10 and perform a new measurement when the physiological characteristics are determined to be constant or substantially constant. In this case, a new physiological characteristic measurement by the first blood pressure measurement device 8 may also be required since measurements are required by both devices 8, 10 simultaneously (or generally at the same time).

According to the present invention, after step 105, the control unit 4 is adapted to analyze the
physiological characteristic measurement made by the first blood pressure measurement device 8 to determine if the blood pressure has stabilized (i.e. is constant again) after the change in the ventilator 12 settings. Thus, after the ventilator 12 settings are changed in order to induce the change in the blood pressure, the control unit 4 waits until the change in blood pressure has stabilized or 'plateaued' before initiating steps 107 and 109. The control unit 4 can determine whether the blood pressure has stabilized or plateaued in the same way as describe above for checking whether a blood pressure measurement by the second blood pressure measurement device 10 is reliable. For example, with reference to Fig. 1, the control unit 4 can delay the triggering of steps 107 and 109 until around time T=1 1 seconds or 12 seconds when the blood pressure has stabilized.

The method in Fig. 5 can be performed when the first blood pressure measurement device 8 is first activated. In some embodiments, the method in Fig. 5 can be repeated periodically to determine a new or updated calibration parameter. Alternatively the method in Fig. 5 can be repeated after a certain time has elapsed since the calibration parameter was determined. As another alternative, the method in Fig. 5 can be repeated when features or characteristics in the surrogate measurement (i.e. the measurement of the physiological characteristic that is used to determine the blood pressure) have changed considerably. For example, the method in Fig. 5 can be repeated when the difference of a feature or physiological characteristic measurement compared with its value from the time of the last calibration measurement exceeds a defined threshold. In a specific example, in the case of a PPG signal, a considerable change can be considered as a change in the amplitude (i.e. the AC value) of the PPG signal of a factor of two or more. In another specific example, in the case of a PPG signal, a considerable change can be considered as a change in the DC value of the PPG signal of a factor of two or more. As another alternative, the method in Fig. 5 can be repeated when the difference between a blood pressure measurement determined from the physiological characteristic measurement compared with a blood pressure measurement determined from a physiological characteristic measurement obtained just after the calibration parameter was determined (e.g. the third physiological characteristic measurement) exceeds a defined threshold. In yet another alternative, measurements from another sensor that measures a physiological characteristic of the subject can be used to determine if a recalibration needs to be performed. The physiological characteristic can be heart rate, the presence of arrhythmia, a change in fluid volume, etc.

An exemplary implementation of the method of Fig. 5 with reference to the apparatus 2, devices 8, 10 and ventilator 12 shown in Fig. 4 is described below. The PPG sensor 32 and ECG electrodes 34 that form part of the first blood pressure measurement device 8 are used to continuously obtain measurements of blood pressure.

When it is time to perform a calibration (for example because a certain amount of time has elapsed after the previous calibration, because features in the surrogate physiological characteristic have changed considerably, or because one or more other sensors have measured a considerable change), the arm cuff 36 is inflated and deflated to obtain an oscillometric blood pressure measurement to determine the 'real' or 'actual' blood pressure (step 103). The time window in which this blood pressure measurement is obtained is referred to as t1. A physiological characteristic measurement is also obtained by the first blood pressure measurement device 8 in time t1 (step 101). After that, the control unit 4 controls the ventilator 12 to change its flow and/or pressure to induce a blood pressure change in the subject 30 (step 105). When this blood pressure change has been effected and the blood pressure has stabilized or plateaued (or is expected to stabilize or plateau, for example based on previous observations of the reaction of the subject's blood pressure to the change by the ventilator 12, or observations for a population of subjects), the second blood pressure measurement device 10 obtains a further 'real' blood pressure measurement using the arm cuff 36 (step 109). The time window in which this blood pressure measurement is obtained is referred to as t2. A further measurement of the physiological characteristic is also obtained by the first blood pressure measurement device 8 in this time window (step 107).

The control unit 4 can perform a check to determine if the first and second blood pressure measurements obtained using the arm cuff 36 took place in time windows where the blood pressure level was approximately constant, by checking if the surrogate measurement (e.g. PAT, PTT, or features in the PPG signal) was approximately constant in these periods. If so, at least one physiological characteristic measurement by the first blood pressure measurement device 8 obtained during t1 (or optionally a plurality of blood pressure measurements obtained by the first blood pressure measurement device 8 during t1 that are averaged) is paired with the first blood pressure measurement obtained by the second blood pressure measurement device 10 in t1, and at least one physiological characteristic measurement by the first blood pressure measurement device 8 obtained during t2 (or optionally a plurality of physiological characteristic measurements obtained by the first blood pressure measurement device 8 during t2 that are averaged) is paired with the second blood pressure measurement obtained by the second blood pressure measurement device 10 in t2. Subsequently, a relationship is determined between each of the surrogate physiological characteristic measurements and the corresponding actual blood pressure (step 111). This relationship provides the one or more calibration parameters that will be used for the continuous monitoring of the blood pressure using the first blood pressure measurement device 8 until the next calibration takes place.

In embodiments where the effect of a Valsalva manoeuvre is induced by the ventilator 12 (e.g. by closing a valve during one exhalation), the timing of the second physiological characteristic measurement and the second blood pressure measurement (steps 107 and 109) becomes quite critical. As shown in Fig. 1, in healthy people, the blood pressure drop typically has a plateau between 10 and 23 seconds. For good oscillometric blood pressure measurements, measurements over a number of heart beats are required in the measurement time window and the blood pressure level also needs to be relatively stable in this time window. Preferably there are at least five heart beats in the measurement time window. However, especially when there is no good estimation of the blood pressure level available beforehand, it is not uncommon that as many as fifteen heart beats are needed in the time window. This means that, at a heart rate of 60 beats per minute (bpm), the time window needs to be at least four (but often more) seconds long. These heart beats need to fall in the period of the plateau. Thus, this requires proper timing of when to start inflating and/or deflating the cuff 36 (i.e. proper timing of when to obtain the second blood pressure measurement (step 109)). In subjects with a disturbed autonomic nervous system (e.g. due to their illness or medication), there might be a shorter plateau period, or even no plateau at all after a Valsalva manoeuvre, or it could have a different timing (e.g. start later, etc.). For that reason, the check in stability of the surrogate measurement during time window t2 is performed. In the case of no stability, the second calibration point (i.e. the second blood pressure measurement) cannot be relied upon.

In some embodiments, the control unit 4 can observe the stability of the surrogate physiological characteristic measurement after each change in air flow by the ventilator 12 and determine for how long the blood pressure level is stable after the change. The control unit 4 can then use that information to determine the timing of the triggering of the second blood pressure measurement (step 109).

One way to minimize the problems with the timing of the second blood pressure measurement is to change to the air flow in step 105 and to hold the ventilator 12 at those settings until the blood pressure level stabilizes (i.e. instead of applying a short-term change). When this stability or plateau has been reached, the real blood pressure measurement with the arm cuff 36 can be performed. After the real blood pressure measurement has been measured, the control unit 4 can control the ventilator 12 to change back to its former settings. As an example, suppose that a ventilator 12 is used with, in its normal situation, a continuous positive airway pressure of 4 cmH2O. The first calibration measurement (i.e. the first blood pressure measurement in step 103) is obtained while the pressure has been 4 cmH2O for some time. In the case of positive airway pressure for treating sleep apnea, the pressure could be 4 cmH2O for several hours. To induce a blood pressure change, the pressure is increased, for example to 12 cmH2O, and this pressure is maintained until the second calibration measurement (step 109) has been performed (which is only triggered as soon as the surrogate measurement has become stable). After that, the ventilator 12 can be controlled to return the positive airway pressure to 4 cmH2O. Instead of triggering the second calibration measurement by stability of the (features in the) surrogate measurement, the control unit 4 can alternatively wait a certain set time after the ventilator 12 change (e.g. always 30 seconds), when the blood pressure is expected to have reached a stable level.

It will be appreciated that although the method in Fig. 5 only requires a single measurement by each blood pressure measurement device 8, 10 before and after the induced blood pressure change, it would be possible to perform the calibration using further pairs of physiological characteristic measurements and blood pressure measurements. In this case, the method could comprise a further instance of step 105 where the air flow from the ventilator 12 is further changed to induce a different blood pressure change, and then steps 107 and 109 can be performed at that different blood pressure. For example in the CPAP example above, the positive airway pressure can be changed from the normal setting of 4 cmH2O to 12 cmH2O to induce a blood pressure change, and then from 12 cmH2O to 8 cmH2O to induce a different change. After the third set of blood pressure measurements are obtained the ventilator 12 can be controlled to return the positive pressure back to 4 cmH20.

It will be appreciated that when the change(s) in ventilator 12 settings give rise to two blood pressure levels that follow each other quite closely in time, and also when the timing of cuff inflation and deflation is selected properly, it is possible to obtain the first calibration measurement (step 103) during inflation of the cuff 36 and obtain the second calibration measurement (step 109) during deflation of the cuff 36.

There is therefore provided an improved method and apparatus for calibrating measurements of blood pressure obtained by a blood pressure measurement device.

While the invention has been illustrated and described in detail in the drawings and foregoing description, such illustration and description are to be considered illustrative or exemplary and not restrictive; the invention is not limited to the disclosed embodiments.

Variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other processing unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. An apparatus (2) for determining a calibration parameter for a first blood pressure, BP, measurement device (8), the apparatus (2) comprising:
a control unit (4) that is to be coupled to a first BP measurement device (8) that is for obtaining physiological characteristic measurements of a physiological characteristic of a subject (30) and for determining a blood pressure measurement of the subject (30) from the physiological characteristic measurements, a second BP measurement device (10) that is for obtaining measurements of the blood pressure of the subject (30) and a ventilator (12) that is for providing a controlled flow of gas to the subject (30), wherein the control unit (4) is configured to:
obtain a first physiological characteristic measurement of the subject (30) using the first BP measurement device (8);
control the second BP measurement device (10) to obtain a first BP measurement of the subject (30);
control the ventilator (12) to change one or more properties of the flow of gas to the subject (30), and thereby change the BP of the subject (30);
analyze a signal from the first BP measurement device (8) to determine when the BP is stable following the change of the one or more properties of the flow of gas;
once the BP is determined to be stable following the change of the one or more properties of the flow of gas, obtain a second physiological characteristic measurement of the subject (30) using the first BP measurement device (8);
once the BP is determined to be stable following the change of the one or more properties of the flow of gas, control the second BP measurement device (10) to obtain a second BP measurement of the subject (30); and
determine a calibration parameter for determining BP measurements from physiological characteristic measurements obtained by the first blood pressure measurement device (8) from an analysis of the first physiological characteristic measurement, the second physiological characteristic measurement, the first BP measurement and the second BP measurement.

2. An apparatus (2) as claimed in claim 1, wherein the second BP measurement device (10) comprises first and second inflatable cuffs (36) for use on different limbs of the subject (30), and wherein the control unit (4) is configured to control the second BP measurement device (10) to obtain the first BP measurement of the subject (30) using the first inflatable cuff (36) and to control the second BP measurement device (10) to obtain the second BP measurement of the subject (30) using the second inflatable cuff.

3. An apparatus (2) as claimed in claim 1 or 2, wherein the control unit (4) is further configured to:
obtain a third BP measurement of the subject (30) from a third physiological characteristic measurement by the first BP measurement device (8) and the determined calibration parameter.

4. An apparatus (2) as claimed in any of claims 1-3, wherein the control unit (4) is configured to control the ventilator (12) to change one or more properties of the flow of gas to the subject (3) to:
(i) change the composition of the flow of gas to the subject (30); or
(ii) induce a sigh in the subject (30); or
(iii) induce a Valsalva manoeuvre in the subject (30); or
(iv) induce a Mueller manoeuvre in the subject (30).

5. A system comprising:
the apparatus (2) as claimed in any of claims 1-4;
the first blood pressure measurement device (8);
the second blood pressure measurement device (10); and
the ventilator (12).

6. A computer implemented method of determining a calibration parameter for a first blood pressure measurement device, the method comprising:
obtaining (101) a first physiological characteristic measurement of a subject using the first BP measurement device, wherein the first BP measurement device is for obtaining physiological characteristic measurements of a physiological characteristic of the subject and for determining a blood pressure measurement of the subject from the physiological characteristic measurements;
controlling (103) a second BP measurement device to obtain a first BP measurement of the subject;
controlling (105) a ventilator to change one or more properties of a flow of gas to the subject, and thereby change the BP of the subject;
analyzing a signal from the first BP measurement device to determine when the BP is stable following the change of the one or more properties of the flow of gas;
once the BP is determined to be stable following the change of the one or more properties of the flow of gas, obtaining (107) a second physiological characteristic measurement of the subject using the first BP measurement device;
once the BP is determined to be stable following the change of the one or more properties of the flow of gas, controlling (109) the second BP measurement device to obtain a second BP measurement of the subject; and
determining (111) a calibration parameter for determining BP measurements from physiological characteristic measurements obtained by the first blood pressure measurement device from an analysis of the first physiological characteristic measurement, the second physiological characteristic measurement, the first BP measurement and the second BP measurement.

7. A method as defined in claim 6, wherein the steps of obtaining (101) the first physiological characteristic measurement and controlling (103) the second BP measurement device to obtain the first BP measurement are performed at generally the same time.

8. A method as defined in claim 6 or 7, wherein the steps of obtaining (107) the second physiological characteristic measurement and controlling (109) the second BP measurement device to obtain the second BP measurement are performed at generally the same time.

9. A method as defined in claim 6, 7 or 8, wherein the second BP measurement device comprises first and second inflatable cuffs for use on different limbs of the subject, and
wherein the step of controlling (103) the second BP measurement device to obtain the first BP measurement comprises controlling the second BP measurement device to obtain the first BP measurement of the subject using the first inflatable cuff, and
wherein the step of controlling (109) the second BP measurement device to obtain the second BP measurement comprises controlling the second BP measurement device to obtain the second BP measurement of the subject using the second inflatable cuff.

10. A method as defined in any of claims 6-9, wherein the method further comprises the step of:
obtaining (113) a third BP measurement of the subject from a third physiological characteristic measurement by the first BP measurement device and the determined calibration parameter.

11. A method as claimed in any of claims 6-10, wherein the step of controlling (105) a ventilator to change one or more properties of a flow of gas to the subject comprises controlling the ventilator to do one of:
(i) change the composition of the flow of gas to the subject;
(ii) induce a sigh in the subject;
(iii) induce a Valsalva manoeuvre in the subject; or
(iv) induce a Mueller manoeuvre in the subject.

12. A computer program product comprising a computer readable medium having computer readable code embodied therein, the computer readable code being configured such that, on execution by the control unit of the apparatus according to claim 1, the apparatus according to claim 1 is caused to perform the method of any of claims 6-11.

## Patentansprüche

1. Vorrichtung (2) zum Bestimmen eines Kalibrierungsparameters für eine erste Blutdruck(BP)-Messvorrichtung (8), wobei die Vorrichtung (2) umfasst:
eine Steuereinheit (4), die mit einer ersten BP-Messvorrichtung (8) zu koppeln ist, die dient, um Messungen der physiologischen Eigenschaft einer physiologischen Eigenschaft einer Person (30) zu erhalten und eine Blutdruckmessung der Person (30) aus den Messungen der physiologischen Eigenschaft zu bestimmen, eine zweite BP-Messvorrichtung (10), die dient, um Messungen des Blutdrucks der Person (30) zu erhalten, und ein Beatmungsgerät (12), das dient, um der Person (30) einen kontrollierten Gasstrom bereitzustellen, wobei die Steuereinheit (4) konfiguriert ist, um:
Erhalten einer ersten Messung der physiologischen Eigenschaft der Person (30) unter Verwendung der ersten BP-Messvorrichtung (8);
Steuern der zweiten BP-Messvorrichtung (10), um eine erste BP-Messung der Person (30) zu erhalten;
Steuern des Beatmungsgeräts (12), um eine oder mehrere Eigenschaften des Gasstroms zu der Person (30) zu ändern und dadurch den BP der Person (30) zu ändern;
Analysieren eines Signals von der ersten BP-Messvorrichtung (8), um zu bestimmen, wenn der BP nach der Änderung der einen oder mehreren Eigenschaften des Gasstroms stabil ist;
sobald bestimmt wird, dass der BP nach der Änderung der einen oder mehreren Eigenschaften des Gasstroms stabil ist, Erhalten einer zweiten Messung der physiologischen Eigenschaft der Person (30) unter Verwendung der ersten BP-Messvorrichtung (8);
sobald bestimmt wird, dass der BP nach der Änderung der einen oder mehreren Eigenschaften des Gasstroms stabil ist, Steuern der zweiten BP-Messvorrichtung (10), um eine zweite BP-Messung der Person (30) zu erhalten; und
Bestimmen eines Kalibrierungsparameters zum Bestimmen von BP-Messungen aus Messungen der physiologischen Eigenschaft, die von der ersten Blutdruckmessvorrichtung (8) aus einer Analyse der ersten Messung der physiologischen Eigenschaft, der zweiten Messung der physiologischen Eigenschaft, der ersten BP-Messung und der zweiten BP-Messung erhalten werden.

2. Vorrichtung (2) nach Anspruch 1, wobei die zweite BP-Messvorrichtung (10) erste und zweite aufblasbare Manschetten (36) zur Verwendung an verschiedenen Gliedmaßen der Person (30) umfasst und wobei die Steuereinheit (4) konfiguriert ist, um die zweite BP-Messvorrichtung (10) zu steuern, um die erste BP-Messung der Person (30) unter Verwendung der ersten aufblasbaren Manschette (36) zu erhalten, und die zweite BP-Messvorrichtung (10) zu steuern, um die zweite BP-Messung der Person (30) unter Verwendung der zweiten aufblasbaren Manschette zu erhalten.

3. Vorrichtung (2) nach Anspruch 1 oder 2, wobei die Steuereinheit (4) ferner konfiguriert ist, zum:
Erhalten einer dritten BP-Messung der Person (30) aus einer dritten Messung der physiologischen Eigenschaft durch die erste BP-Messvorrichtung (8) und dem bestimmten Kalibrierungsparameter.

4. Vorrichtung (2) nach einem der Ansprüche 1 bis 3, wobei die Steuereinheit (4) konfiguriert ist, um das Beatmungsgerät (12) zu steuern, um eine oder mehrere Eigenschaften des Gasstroms zu der Person (3) zu ändern, zum:
(i) Ändern der Zusammensetzung des Gasstroms zu der Person (30); oder
(ii) Induzieren eines Seufzens in der Person (30);
oder
(iii) Induzieren eines Valsalva-Manövers in der Person (30); oder
(iv) Induzieren eines Mueller-Manövers in der Person (30).

5. System, umfassend:
die Vorrichtung (2) nach einem der Ansprüche 1 bis 4;
die erste Blutdruckmessvorrichtung (8);
die zweite Blutdruckmessvorrichtung (10); und
das Beatmungsgerät (12).

6. Computerimplementiertes Verfahren zum Bestimmen eines Kalibrierungsparameters für eine erste Blutdruckmessvorrichtung, wobei das Verfahren umfasst:
Erhalten (101) einer ersten Messung der physiologischen Eigenschaft einer Person unter Verwendung der ersten BP-Messvorrichtung, wobei die erste BP-Messvorrichtung zum Erhalten von Messungen der physiologischen Eigenschaft einer physiologischen Eigenschaft der Person und zum Bestimmen einer Blutdruckmessung der Person aus den Messungen der physiologischen Eigenschaft;
Steuern (103) einer zweiten BP-Messvorrichtung, um eine erste BP-Messung der Person zu erhalten;
Steuern (105) eines Beatmungsgeräts, um eine oder mehrere Eigenschaften eines Gasstroms zu der Person zu ändern, und dadurch den BP der Person zu ändern;
Analysieren eines Signals von der ersten BP-Messvorrichtung, um zu bestimmen, wenn der BP nach der Änderung der einen oder mehreren Eigenschaften des Gasstroms stabil ist;
sobald bestimmt wird, dass der BP nach der Änderung der einen oder mehreren Eigenschaften des Gasstroms stabil ist, Erhalten (107) einer zweiten Messung der physiologischen Eigenschaft der Person unter Verwendung der ersten BP-Messvorrichtung;
sobald bestimmt wird, dass der BP nach der Änderung der einen oder mehreren Eigenschaften des Gasstroms stabil ist, Steuern (109) der zweiten BP-Messvorrichtung, um eine zweite BP-Messung der Person zu erhalten; und
Bestimmen (111) eines Kalibrierungsparameters zum Bestimmen von BP-Messungen aus Messungen der physiologischen Eigenschaft, die von der ersten Blutdruckmessvorrichtung aus einer Analyse der ersten Messung der physiologischen Eigenschaft, der zweiten Messung der physiologischen Eigenschaft, der ersten BP-Messung und der zweiten BP-Messung erhalten werden.

7. Verfahren nach Anspruch 6, wobei die Schritte des Erhaltens (101) der ersten Messung der physiologischen Eigenschaft und des Steuerns (103) der zweiten BP-Messvorrichtung zum Erhalten der ersten BP-Messung im Allgemeinen gleichzeitig durchgeführt werden.

8. Verfahren nach Anspruch 6 oder 7, wobei die Schritte des Erhaltens (107) der zweiten Messung der physiologischen Eigenschaft und des Steuerns (109) der zweiten BP-Messvorrichtung zum Erhalten der zweiten BP-Messung im Allgemeinen gleichzeitig durchgeführt werden.

9. Verfahren nach Anspruch 6, 7 oder 8, wobei die zweite BP-Messvorrichtung erste und zweite aufblasbare Manschetten zur Verwendung an verschiedenen Gliedmaßen der Person umfasst, und
wobei der Schritt des Steuerns (103) der zweiten BP-Messvorrichtung, um die erste BP-Messung zu erhalten, das Steuern der zweiten BP-Messvorrichtung umfasst, um die erste BP-Messvorrichtung der Person unter Verwendung der ersten aufblasbaren Manschette zu erhalten, und
wobei der Schritt des Steuerns (109) der zweiten BP-Messvorrichtung, um die zweite BP-Messung zu erhalten, das Steuern der zweiten BP-Messvorrichtung umfasst, um die zweite BP-Messvorrichtung der Person unter Verwendung der zweiten aufblasbaren Manschette zu erhalten.

10. Verfahren nach einem der Ansprüche 6 bis 9, wobei das Verfahren ferner den folgenden Schritt umfasst:
Erhalten (113) einer dritten BP-Messung der Person aus einer dritten Messung der physiologischen Eigenschaft durch die erste BP-Messvorrichtung und dem bestimmten Kalibrierungsparameter.

11. Verfahren nach einem der Ansprüche 6 bis 10, wobei der Schritt des Steuerns (105) eines Beatmungsgeräts, um eine oder mehrere Eigenschaften eines Gasstroms zu der Person zu ändern, das Steuern des Beatmungsgeräts umfasst, um eines der folgenden auszuführen:
(i) Ändern der Zusammensetzung des Gasstroms zu der Person;
(ii) Induzieren eines Seufzens in der Person;
(iii) Induzieren eines Valsalva-Manövers in der Person; oder
(iv) Induzieren eines Mueller-Manövers in der Person.

12. Computerprogrammprodukt, das ein computerlesbares Medium mit darin enthaltenem computerlesbaren Code umfasst, wobei der computerlesbare Code so konfiguriert ist, dass bei Ausführung durch die Steuereinheit der Vorrichtung nach Anspruch 1 veranlasst wird, dass die Vorrichtung nach Anspruch 1 das Verfahren nach einem der Ansprüche 6 bis 11 durchführt.

## Revendications

1. Appareil (2) pour déterminer un paramètre d'étalonnage pour un premier dispositif (8) de mesure de la pression sanguine, BP, l'appareil (2) comprenant:
une unité de commande (4) qui doit être couplée à un premier dispositif de mesure de BP (8) qui est destiné à obtenir des mesures de caractéristique physiologique d'une caractéristique physiologique d'un sujet (30) et à déterminer une mesure de la pression sanguine du sujet (30) à partir des mesures de la caractéristique physiologique, un deuxième dispositif de mesure de BP (10) qui est destiné à obtenir des mesures de la pression sanguine du sujet (30) et un ventilateur (12) qui est destiné à fournir un flux de gaz commandé au sujet (30), où l'unité de commande (4) est configurée pour:
obtenir une première mesure d'une caractéristique physiologique du sujet (30) à l'aide du premier dispositif de mesure de BP (8);
commander le deuxième dispositif de mesure de BP (10) pour obtenir une première mesure de BP du sujet (30) ;
commander le ventilateur (12) pour modifier une ou plusieurs propriétés du flux de gaz vers le sujet (30), et ainsi modifier la BP du sujet (30);
analyser un signal provenant du premier dispositif de mesure de BP (8) pour déterminer quand la BP est stable suite au changement de l'une ou des plusieurs propriétés du flux de gaz;
une fois que la BP est déterminée comme étant stable suite au changement de l'une ou des plusieurs propriétés du flux de gaz, obtenir une deuxième mesure d'une caractéristique physiologique du sujet (30) à l'aide du premier dispositif de mesure de BP (8);
une fois que la BP est déterminée comme étant stable suite au changement de l'une ou des plusieurs propriétés du flux de gaz, commander le deuxième dispositif de mesure de BP (10) pour obtenir une deuxième mesure de BP du sujet (30); et
déterminer un paramètre d'étalonnage pour déterminer des mesures de BP à partir des mesures d'une caractéristique physiologique obtenues par le premier dispositif de mesure de la pression sanguine (8) à partir d'une analyse de la première mesure de la caractéristique physiologique, de la deuxième mesure de la caractéristique physiologique, de la première mesure de BP et de la deuxième mesure de BP.

2. Appareil (2) tel que revendiqué dans la revendication 1, dans lequel le deuxième dispositif de mesure de BP (10) comprend des premier et deuxième brassards gonflables (36) destinés à être utilisés sur différents membres du sujet (30), et dans lequel l'unité de commande (4) est configurée pour commander le deuxième dispositif de mesure de BP (10) pour obtenir la première mesure de BP du sujet (30) à l'aide du premier brassard gonflable (36) et pour commander le deuxième dispositif de mesure de BP (10) pour obtenir la deuxième mesure de BP du sujet (30) à l'aide du deuxième brassard gonflable.

3. Appareil (2) tel que revendiqué dans la revendication 1 ou 2, dans lequel l'unité de commande (4) est configurée en outre pour:
obtenir une troisième mesure de BP du sujet (30) à partir d'une troisième mesure d'une caractéristique physiologique par le premier dispositif de mesure de BP (8) et à partir du paramètre d'étalonnage déterminé.

4. Appareil (2) tel que revendiqué dans l'une quelconque des revendications 1 à 3, dans lequel l'unité de commande (4) est configurée pour commander le ventilateur (12) pour modifier une ou plusieurs propriétés du flux de gaz vers le sujet (3) pour:
(i) modifier la composition du flux de gaz vers le sujet (30); ou
(ii) induire un soupir chez le sujet (30); ou
(iii) induire une manœuvre de Valsalva chez le sujet (30); ou
(iv) induire une manœuvre de Mueller chez le sujet (30) .

5. Système comprenant:
l'appareil (2) tel que revendiqué dans l'une quelconque des revendications 1 à 4;
le premier dispositif de mesure de la pression sanguine (8);
le deuxième dispositif de mesure de la pression sanguine (10); et
le ventilateur (12).

6. Procédé mis en œuvre par ordinateur pour déterminer un paramètre d'étalonnage pour un premier dispositif de mesure de la pression sanguine, le procédé consistant à:
obtenir (101) une première mesure d'une caractéristique physiologique d'un sujet à l'aide du premier dispositif de mesure de BP, où le premier dispositif de mesure de BP est destiné à obtenir des mesures de caractéristique physiologique d'une caractéristique physiologique du sujet et à déterminer une mesure de la pression sanguine du sujet à partir des mesures de la caractéristique physiologique;
commander (103) un deuxième dispositif de mesure de BP pour obtenir une première mesure de BP du sujet;
commander (105) un ventilateur pour modifier une ou plusieurs propriétés d'un flux de gaz vers le sujet, et ainsi modifier la BP du sujet;
analyser un signal provenant du premier dispositif de mesure de BP pour déterminer quand la BP est stable suite au changement de l'une ou des plusieurs propriétés du flux de gaz;
une fois que la BP est déterminée comme étant stable suite au changement de l'une ou des propriétés du flux de gaz, obtenir (107) une deuxième mesure d'une caractéristique physiologique du sujet à l'aide du premier dispositif de mesure de BP;
une fois que la BP est déterminée comme étant stable suite au changement de l'un ou des plusieurs propriétés du flux de gaz, commander (109) le deuxième dispositif de mesure de BP pour obtenir une deuxième mesure de BP du sujet; et
déterminer (111) un paramètre d'étalonnage pour déterminer des mesures de BP à partir de mesures d'une caractéristique physiologique obtenues par le premier dispositif de mesure de pression sanguine à partir d'une analyse de la première mesure de la caractéristique physiologique, de la deuxième mesure de la caractéristique physiologique, de la première mesure de BP et de la deuxième mesure de BP.

7. Procédé tel que défini dans la revendication 6, dans lequel les étapes consistant à obtenir (101) la première mesure de la caractéristique physiologique et commander (103) le deuxième dispositif de mesure de BP pour obtenir la première mesure de BP sont généralement effectuées en même temps.

8. Procédé tel que défini dans la revendication 6 ou 7, dans lequel les étapes consistant à obtenir (107) la deuxième mesure de la caractéristique physiologique et commander (109) le deuxième dispositif de mesure de BP pour obtenir la deuxième mesure de BP sont effectuées généralement en même temps.

9. Procédé tel que défini dans la revendication 6, 7 ou 8, dans lequel le deuxième dispositif de mesure de BP comprend des premier et deuxième brassards gonflables destinés à être utilisés sur différents membres du sujet, et
dans lequel l'étape consistant à commander (103) le deuxième dispositif de mesure de BP pour obtenir la première mesure de BP consiste à commander le deuxième dispositif de mesure de BP pour obtenir la première mesure de BP du sujet à l'aide du premier brassard gonflable, et
dans lequel l'étape consistant à commander (109) le deuxième dispositif de mesure de BP pour obtenir la deuxième mesure de BP consiste à commander le deuxième dispositif de mesure de BP pour obtenir la deuxième mesure de BP du sujet à l'aide du deuxième brassard gonflable.

10. Procédé tel que défini dans l'une quelconque des revendications 6 à 9, dans lequel le procédé en outre comprend l'étape consistant à:
obtenir (113) une troisième mesure de BP du sujet à partir d'une troisième mesure d'une caractéristique physiologique par le premier dispositif de mesure de BP et à partir du paramètre d'étalonnage déterminé.

11. Procédé tel que revendiqué dans l'une quelconque des revendications 6 à 10, dans lequel l'étape consistant à commander (105) un ventilateur pour modifier une ou plusieurs propriétés d'un flux de gaz vers le sujet consiste à commander le ventilateur pour effectuer l'une des opérations suivantes:
(i) modifier la composition du flux de gaz vers le sujet;
(ii) induire un soupir chez le sujet;
(iii) induire une manœuvre de Valsalva chez le sujet; ou
(iv) induire une manœuvre de Mueller chez le sujet.

12. Produit de programme informatique comprenant un support lisible par ordinateur ayant un code lisible par ordinateur incorporé dans celui-ci, le code lisible par ordinateur étant configuré de telle sorte que, lors de l'exécution par l'unité de commande de l'appareil selon la revendication 1, l'appareil selon la revendication 1 est amené à exécuter le procédé selon l'une quelconque des revendications 6 à 11.
